(19) Europäisches Patentamt
European Patent Office
Office européen des brevets

(11) EP 2 377 861 B1

(12) # EUROPÄISCHE PATENTSCHRIFT

(45) Veröffentlichungstag und Bekanntmachung des Hinweises auf die Patenterteilung:
**04.12.2013 Patentblatt 2013/49**

(21) Anmeldenummer: **10004105.2**

(22) Anmeldetag: **17.04.2010**

(51) Int Cl.:
*C07D 401/14* (2006.01)
*C22B 60/02* (2006.01)
*G21F 9/12* (2006.01)
*C22B 60/04* (2006.01)
*C01G 56/00* (2006.01)
*G21C 19/46* (2006.01)
*C01F 17/00* (2006.01)

(54) **Wasserlösliche Bis-triazinyl-pyridine, -bipyridine und terpyridine, deren Synthese und Verwendung**

Water-soluble bis-triazinyl-pyridines, bipyridines and terpyridines, synthesis and use of same

Bis-triazinyle-pyridines, bipyridines et terpyridines solubles dans l'eau, leur synthèse et leur utilisation

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(43) Veröffentlichungstag der Anmeldung:
**19.10.2011 Patentblatt 2011/42**

(73) Patentinhaber: **Karlsruher Institut für Technologie
76131 Karlsruhe (DE)**

(72) Erfinder:
- **Müllich, Udo
76344 Eggenstein-Leopoldshafen (DE)**
- **Geist, Andreas
76344 Eggenstein-Leopoldshafen (DE)**
- **Zevaco, Thomas
76344 Eggenstein-Leopoldshafen (DE)**

(74) Vertreter: **Gärtner, Stephan
Karlsruher Institut für Technologie
Innovationsmanagement
Postfach 36 40
76021 Karlsruhe (DE)**

(56) Entgegenhaltungen:
**EP-A1- 0 070 226 DE-C1- 19 810 895**

- **TRAISTER, GEORGE L. ET AL: "Water-soluble sulfonated chromogenic reagents of the ferroin type and determination of iron and copper in water, blood serum, and beer with the tetraammonium salt of 2,4-bis(5,6-diphenyl-1,2,4-triazin-3-yl)py ridinetetrasulfonic acid" ANALYTICAL CHEMISTRY , 48(8), 1216-20 CODEN: ANCHAM; ISSN: 0003-2700, 1976, XP002589534**
- **CRYBERG, RICHARD L. ET AL: "Sulfonation of bathophenanthroline and bathocuproine" PROCEEDINGS OF THE IOWA ACADEMY OF SCIENCE , 70, 184-7 CODEN: PIAIA9; ISSN: 0085-2236, 1963, XP009135493**
- **NILSSON M ET AL: "Review article: A review of the development and operational characteristics of the TALSPEAK process" SOLVENT EXTRACTION AND ION EXCHANGE NOVEMBER 2007 TAYLOR AND FRANCIS INC. US, Bd. 25, Nr. 6, November 2007 (2007-11), Seiten 665-701, XP0009135215 DOI: DOI: 10.1080/07366290701634636**
- **MODOLO G ET AL: "SYNERGISTIC SELECTIVE EXTRACTION OF ACTINIDES(III) OVER LANTHANIDES FROM NITRIC ACID USING NEW AROMATIC DIORGANYLDITHIOPHOSPHINIC ACIDS AND NEUTRAL ORGANOPHOSPHORUS COMPOUNDS" SOLVENT EXTRACTION AND ION EXCHANGE, DEKKER, NEW YORK, NY, US, Bd. 17, Nr. 1, 1. Januar 1999 (1999-01-01) , Seiten 33-53, XP009041958 ISSN: 0736-6299**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft Moleküle bzw. Komplexbildner zur selektiven Komplexierung von Actiniden in wässriger Lösung sowie die Verwendung dieser Moleküle bei der Extraktion von Actiniden bei der Rezyklierung von Kernbrennstoffen.

Abgebrannte Kernbrennstoffe enthalten Radionuklide mit teilweise sehr langer Halbwertszeit. Insbesondere die Transurane wie Plutonium, Neptunium, Americium und Curium sind für einen Großteil der Langzeitradiotoxizität verantwortlich. Im Rahmen der Abtrennungs- und Transmutationsstrategie zur Reduktion der Langzeitradiotoxizität abgebrannter Kernbrennstoffe werden Prozesse zur selektiven Extraktion der Transurane Neptunium, Plutonium, Americium und Curium entwickelt. In diesem Zusammenhang stellt die erforderliche Trennung der zumeist dreiwertig vorliegenden Actiniden von den chemisch ähnlichen Lanthaniden den Schlüsselschritt dar.

Im Stand der Technik sind verschiedene Extraktionsmittel für Actiniden bekannt.

DE 198 10 895 C1 beschreibt die Verwendung von alkylierten Bis-triazinyl-pyridinen (BTP), die als lipophile Komplexbildner in der organischen Phase eingesetzt werden. Die BTP-Moleküle haben allerdings den Nachteil, dass sie eine geringe Stabilität gegenüber Salpetersäure aufweisen. Diese Stabilität gegenüber Salpetersäure ist wesentlich, da abgebrannte Kernbrennstäbe bei der Wiederaufarbeitung in heißer Salpetersäure aufgelöst werden. Die BTP-Verbindungen haben den weiteren Nachteil, dass sie nicht ausreichend radiolysestabil sind, was eine Rezyklierung erschwert.

In dem Artikel von A. Geist, C. Hill, G. Modolo, M.R.St.J. Foreman, M. Weigl, K. Gompper, M.J. Hudson, C. Madic, 6,6'-Bis(5,5,8,8-tetramethyl-5,6,7,8-tetrahydro-benzo-[1,2,4]triazin-3-yl)[2,2']bipyridine, an effective extracting agent for the separation of americium(III) and curium(III) from the lanthanides. Solvent Extr. Ion Exch. 2006, 24 (4), 463-483 werden alkylierte Bis-triazinyl-bipyridyle (BTBP) als Komplexbildner für Actiniden vorgestellt. Diese Moleküle weisen jedoch eine zu langsame Extraktionskinetik auf und eine zu geringe Radiolysestabilität auf. Die Verwendung der BTBP ist ähnlich wie die der BTP auf die Komplexierung der Actiniden in der organischen Phase beschränkt.

Modolo und Odoj beschreiben aromatische Dithiophosphinsäuren als Komplexbildner, die in synergistischer Mischung mit Organophosphaten eine gute Auftrennung von Lanthaniden und Actiniden auch bei hoher Salpetersäurekonzentration erreichen (G. Modolo, R. Odoj, Synergistic selective extraction of actinides(III) over lanthanides from nitric acid using new aromatic diorganyldithiophosphinic acids and neutral organophosphorus compounds. Solvent Extr. Ion Exch. 1999, 17 (1), 33-53). Diese haben allerdings den Nachteil, dass aufgrund der zusätzlichen Heteroatome (Phosphor) bei der Thermolyse feste Sekundärabfälle entstehen. Wünschenswert wäre allerdings, dass bei der Verbrennung nur gasförmige Produkte entstehen.

In der europäischen Wiederaufarbeitung nach dem DIAMEX-SANEX-Konzept (**Diam**ide **Ex**traction, **S**elective **A**cti**n**ide **Ex**traction) werden nach Abtrennung von Uran, Plutonium und ggf. Neptunium im sog. PUREX-Prozess (**P**lutonium-**U**ranium **R**ecovery by **Ex**traction) zunächst Actiniden und Lanthaniden gemeinsam aus dem PUREX-Raffinat abgetrennt (DIAMEX-Prozess). Anschließend werden Actiniden von den Lanthaniden getrennt (SANEX-Prozess).

Eine Vereinfachung diese Schemas sieht eine Zusammenlegung der DIAMEX- und der SANEX-Prozesse vor. Dazu werden zunächst, wie im DIAMEX-Prozess, Actiniden und Lanthaniden aus dem PUREX-Raffinat in eine organische Phase extrahiert, aber anders als im DIAMEX-Prozess nicht gemeinsam rückextrahiert. Anschließend werden aus der beladenen organischen Phase selektiv nur die Actiniden rückextrahiert.

Prinzipiell geht dieses Verfahren auf den vor 45 Jahren entwickelten sog. TALSPEAK-Prozess (**T**rivalent **A**ctinide - **L**anthanide **S**eparation by **P**hosphorous Reagent **E**xtraction from **A**queous **K**omplexes) (B. Weaver, F.A. Kappelmann, TALSPEAK: a new method of separating americium and curium from the lanthanides by extraction from an aqueous solution of an aminopolyacetic acid complex with a monoacidic organophosphate or phosphonate. ORNL-3559, Oak Ridge National Laboratory, 1964 und M. Nilsson, K. Nash, A Review of the development and operational characteristics of the TALSPEAK process. Solvent Extr. Ion Exch. 2007, 25 (6), 665-701)) zurück. Dieses Verfahren besitzt einige Nachteile. Die verwendeten selektiven Komplexbildner sind nur in einem Bereich niedriger Säurestärke (etwa pH > 3) effektiv, was die Verwendung von Pufferreagenzien erfordert. Weiterhin müssen bei der Verwendung eines solvatisierenden Extraktionsmittels die Lanthaniden entweder durch Zugabe von Neutralsalz (z.B. Ammoniumnitrat) oder durch Zusatz eines zweiten, in diesem pH-Bereich effektiven Extraktionsmittels in der organischen Phase gehalten werden (C. Heres et al., Proc. GLOBAL 2009, Paris Sept. 2009).

Es wäre also wünschenswert, das aufwändige DIAMEX-SANEX-Verfahren durch eine direkte Rückextraktion der Actiniden aus der organischen Phase des DIAMEX-Raffinats zu vereinfachen. Alternativ könnten die Actiniden durch entsprechend selektive Komplexbildner in wässriger Lösung gehalten werden, bevor man die Lanthaniden mit lipophilen Komplexbildnern in die organische Phase abtrennt. Hierzu bedarf es allerdings neuer hydrophiler Komplexbildner, die im Gegensatz zum TALSPEAK-Prozess, auch bei höherer Säurestärke selektiv komplexieren, sodass der Einsatz von Puffersubstanzen nicht mehr notwendig ist.

Ausgehend hiervon ist es die Aufgabe der Erfindung, neue hydrophile Komplexierungsmittel vorzustellen, welche die Probleme des Stands der Technik überwinden. Insbesondere sollen sie selektiv Actiniden gegen die chemisch

ähnlichen Lanthaniden komplexieren. Ein Herstellungsverfahren für die Komplexierungsmittel wird ebenfalls vorgeschlagen. Darüber hinaus ist es Aufgabe der Erfindung, eine Verwendung dieser Moleküle im Bereich der Actinidenextraktion vorzustellen. Weiterhin ist es Aufgabe der Erfindung selektive Extraktionsverfahren für Actiniden vorzuschlagen, die die Prozessschritte aus dem Stand der Technik reduzieren und vereinfachen.

Die Aufgabe wird durch die in Anspruch 1 beanspruchten Moleküle, deren Herstellungsverfahren nach Anspruch 3 und das Verfahrensprodukt nach Anspruch 6 gelöst. Weitere Lösungen der Aufgabe sind in der Verwendung gemäß Anspruch 7 und dem Extraktionsverfahren nach Anspruch 9 beansprucht. Bevorzugte Ausführungsformen der Moleküle, deren Herstellung und Verwendung sowie des Extraktionsverfahrens sind in den rückbezogenen Ansprüchen beschrieben.

Im Rahmen der vorliegenden Erfindung sind unter der Bezeichnung "Actiniden" insbesondere Actiniden in oxidierter Form zu verstehen. Die Oxidationsstufen der Actiniden, wie sie auch bei der nuklearen Wiederaufarbeitung vorzufinden sind, liegen bei (III), (IV), (V) oder (VI). Bevorzugter Weise liegen sie in der Oxidationsstufe (III) vor.

Die Erfindung umfasst 2,6-Bis(5,6-di(sulfophenyl)-1,2,4-triazin-3-yl)pyridine ($SO_3$-Ph-BTP), 6,6'-Bis(5,6-di(sulfophenyl)-1,2,4-triazin-3-yl)-2,2'-bipyridine ($SO_3$-Ph-BTBP) und /oder 6,6"-Bis(5,6-di(sulfophenyl)-1,2,4-triazin-3-yl)-2,2':6',2"-terpyridine ($SO_3$-Ph-BTTP) nach der allgemeinen Strukturformel (I)

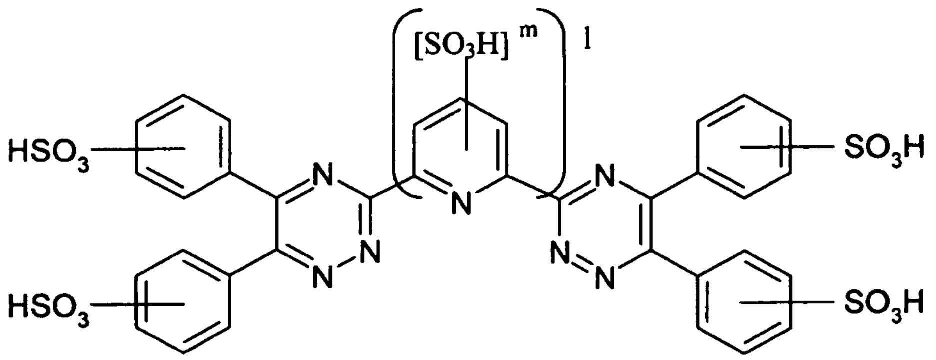

(I),

wobei 1=1,2,3 und m=0,1 ist.

Bei den erfindungsgemäßen $SO_3$-Ph-BTP enthält die Pyridingruppe eine $SO_3H$-Gruppe, bei den $SO_3$-Ph-BTBP hat die Bipyridingruppe keine, eine oder zwei $SO_3H$-Gruppen und bei den $SO_3$-Ph-BTTP enthält die Terpyridingruppe keine, eine, zwei oder drei $SO_3H$-Gruppen.

Konkret umfasst es Pyridine nach der Strukturformel (III), Bipyridine nach den Strukturformeln (IV) bis (VI) und Terpyridine nach der Strukturformel (VII).

Die der Erfindung zugrunde liegenden Verfahren und Verwendungen umfassen auch $SO_3$-Ph-BTP, bei welchen die Pyridingruppe keine $SO_3H$-Gruppe enthält nach der allgemeinen Formel (II). Pyridine gemäß der allgemeinen Formel (II) sind bekannt aus Traister G L, Schilt A, Analytical Chemistry, 48(8), 1216-1220, 1976. Sie wurden dort zur Komplexierung von Eisen- und Kupferionen für analytische Anwendungen verwendet.

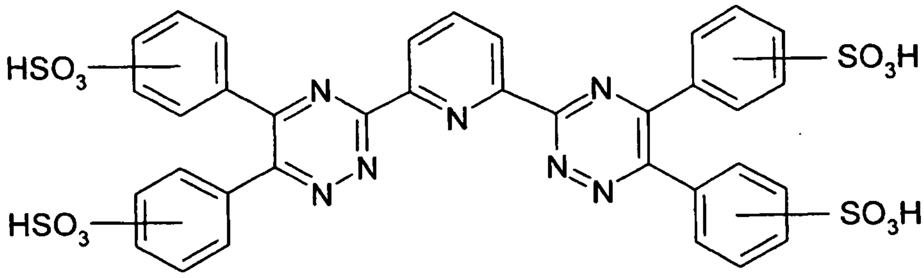

(II)

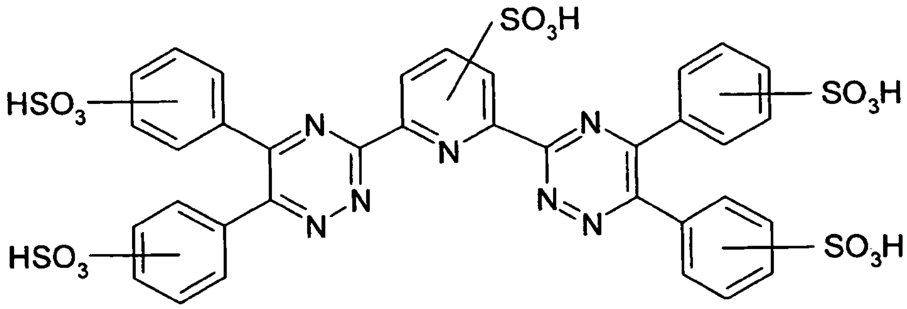

(III)

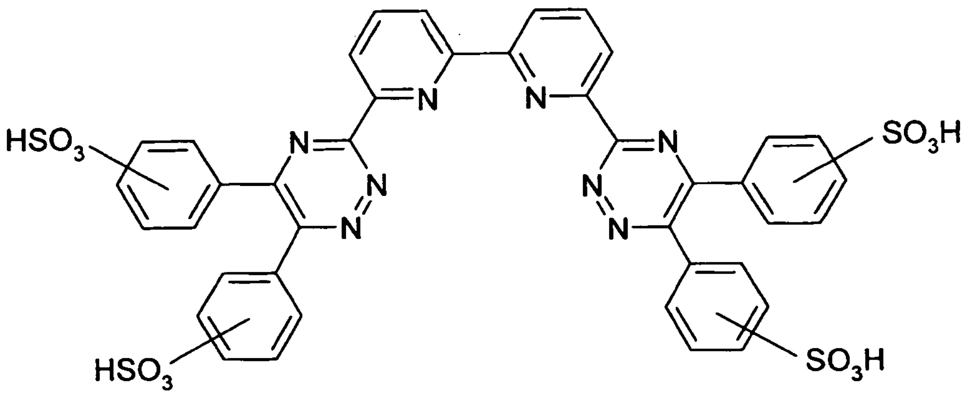

(IV)

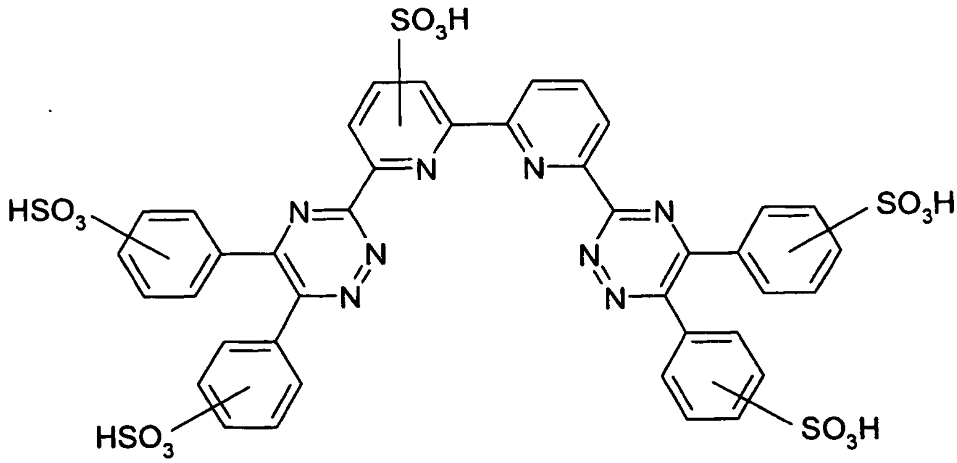

(V)

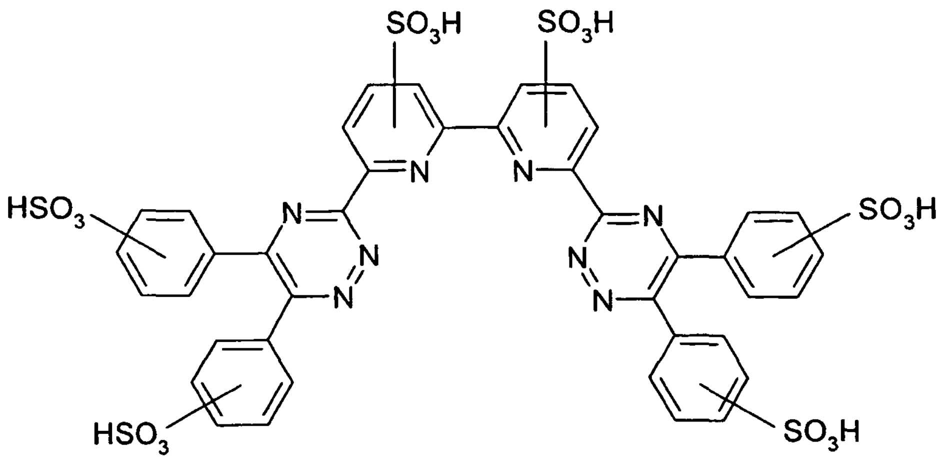

(VI)

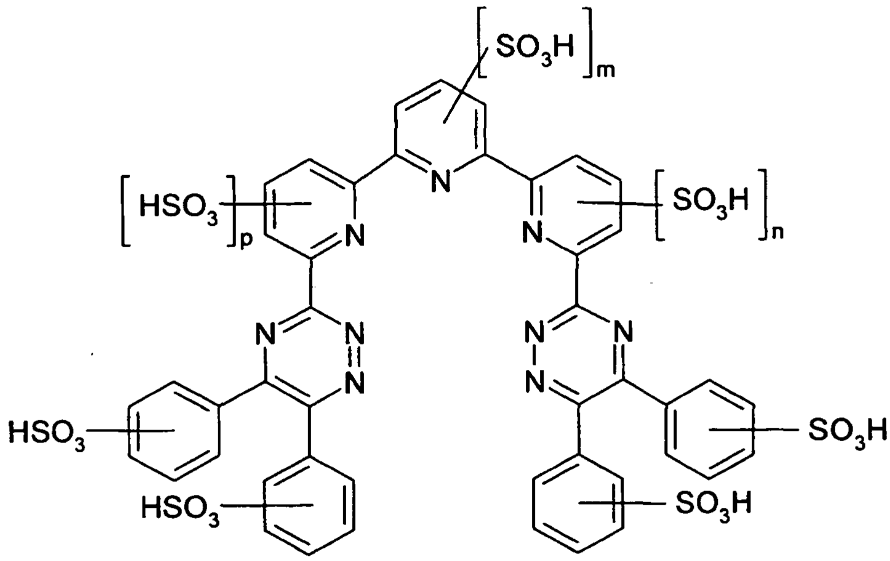

(VII)

p, m, n = 0; 1

In einer bevorzugten Ausführung der erfindungsgemäßen Moleküle befindet sich die Sulfo-Gruppe der Sulfophenylreste in 4-Position. Die Molekülgruppen umfassen demgemäß 2,6-Bis(5,6-di(4-sulfophenyl)-1,2,4-triazin-3-yl) pyridine 6,6'-Bis(5,6-di(4-sulfophenyl)-1,2,4-triazin-3-yl)-2,2'-bipyridine bzw. 6,6''-Bis(5,6-di(4-sulfophenyl)-1,2,4-triazin-3-yl)-2,2':6',2" -terpyridine. In einer weiteren Ausführung sind auch die Pyridinringe, sofern sie eine Sulfogruppe tragen, in 4-Position sulfoniert.

Zur Herstellung der erfindungsgemäßen 2,6-Bis(5,6-di(sulfophenyl)-1,2,4-triazin-3-yl)pyridin ($SO_3$-Ph-BTP), 6,6'-Bis(5,6-di(sulfophenyl)-1,2,4-triazin-3-yl)-2,2'-bipyridin ($SO_3$-Ph-BTBP) und 6,6''-Bis(5,6-di(sulfophenyl)-1,2,4-triazin-3-yl)-2,2':6',2''-terpyridin ($SO_3$-Ph-BTTP) werden die nach dem Stand der Technik hergestellten 2,6-Bis(5,6-diphenyl-1,2,4-triazin-3-yl)pyridin (BTP), 6,6'-Bis(5,6-diphenyl-1,2,4-triazin-3-yl)-2,2'-bipyridin (BTBP) oder 6,6"-Bis(5,6-di(phenyl)-l,2,4-triazin-3-yl)-2,2':6',2''-terpyridin (BTTP) mit einem bekannten Sulfonierungsmittel aus der Gruppe Schwefelsäure, Oleum, Chlorsulfonsäure, Sulfamidsäure oder Schwefeltrioxid umgesetzt. In einer bevorzugten Ausführung der Synthese werden die BTP, BTBP oder BTTP in Oleum umgesetzt. Das Reaktionsgemisch wird vorzugsweise auf 100°C bis 170°C erhitzt und über mehrere Minuten, bevorzugt 5 min - 30 min, bei dieser Temperatur gerührt. Fakultativ kann das Reaktionsgemisch zwischenzeitlich auch über eine mehrtägige, bevorzugt zweitägige, Phase bei Raumtemperatur ruhen.

Die Reaktion kann beispielsweise durch Entfernen von überschüssigen $SO_3$ und anschließender Neutralisierung beendet werden. In einer bevorzugten Ausführung der Synthese wird das Rohprodukt, das bei dieser Synthese erhalten wird, mit einem polaren Lösungsmittel extrahiert. Besonders bevorzugt ist das polare Lösungsmittel Methanol. Typischer weise erhält man somit ein Produktgemisch aus unterschiedlich sulfonierten $SO_3$-Ph-BTP, $SO_3$-Ph-BTBP oder $SO_3$-Ph-BTTP, die entweder nach einer dem Fachmann bekannten Methode, z.B. chromatographisch, durch Umkristallieren etc., aufgetrennt werden kann. Das Rohprodukt, das extrahiert wurde, kann auch direkt als Produktgemisch einer weiteren Verwendung zugeführt werden.

Die auf Basis des beschriebenen Verfahrens hergestellten Verbindungen 2,6-Bis(5,6-di(sulfophenyl)-1,2,4-triazin-3-yl)pyridine, 6,6'-Bis(5,6-di(sulfophenyl)-1,2,4-triazin-3-yl)-2,2'-bipyridine und 6,6''-Bis(5,6-di(sulfophenyl)-1,2,4-triazin-3-yl)-2,2':6',2"-terpyridine sind ebenfalls Bestandteil dieser Erfindung.

Die erfindungsgemäßen $SO_3$-Ph-BTP, $SO_3$-Ph-BTBP oder $SO_3$-Ph-BTTP sind gute Liganden, um mit Actiniden selektiv gegenüber Lanthaniden einen wasserlöslichen Chelatkomplex zu bilden. Die Verwendung von $SO_3$-Ph-BTP, $SO_3$-Ph-BTBP oder $SO_3$-Ph-BTTP, sowie das Produktgemisch aus der Synthese als Komplexbildner zur selektiven Komplexierung von Actiniden in wässriger Lösung ist ein weiterer Teil der vorliegenden Erfindung. Bevorzugt ist deren Verwendung in salpetersaurer Lösung mit einer Säurestärke von 0,01 mol/L bis 3 mol/L.

Weiterhin ist eine bevorzugte Verwendung der erfindungsgemäßen Moleküle zur Komplexierung von Actiniden, die beim Wiederaufarbeitungsprozess von Kernbrennstäben anfallen. Hierzu gehören insbesondere Isotope der Actiniden Americium, Curium, Neptunium und/oder Plutonium. Erfindungsgemäße Moleküle sind in diesem Zusammenhang isomerenreine $SO_3$-Ph-BTP, $SO_3$-Ph-BTBP und $SO_3$-Ph-BTTP, als auch Gemische dieser Moleküle bzw. das Rohprodukt der Synthese.

Die Erfindung umfasst weiterhin ein Verfahren zur selektiven Extraktion von Actiniden umfassend folgende Verfahrensschritte:

a) Bereitstellen einer organischen Lösung enthaltend Lanthaniden und Actiniden, die mit Hilfe eines lipophilen Komplexierungsmittels gelöst sind,
b) Zugabe eines erfindungsgemäßen $SO_3$-Ph-BTP, $SO_3$-Ph-BTBP oder $SO_3$-Ph-BTTP bzw. ein Gemisch derselben in wässriger Lösung,
c) Rückextraktion der Actiniden in die wässrige Phase,
d) Abtrennen der wässrigen Phase.

Dieses Verfahren hat den Vorteil, dass der aktuelle DIAMEX-SANEX-Prozess um einen Prozessschritt reduziert wird, da man Lanthaniden und Actiniden nicht gemeinsam in eine wässrige Phase rückextrahieren muss, sondern selektiv die Actiniden mit Hilfe des erfindungsgemäßen Komplexierungsmittels direkt aus dem organischen Extrakt rückextrahiert. Diese selektive Rückextraktion kann in saurer Lösung stattfinden, ohne dass Neutralsalze oder Puffersubstanzen hinzugefügt werden müssen.

Beispiele für in Schritt a) eingesetzte lipophile Komplexierungsmittel sind TODGA (N,N,N',N'-Tetra-n-octyldiglycolamid) oder D2EHPA (Di-(2-ethylhexyl)phosphorsäure). Generell sind aber alle lipophilen Komplexbildner möglich, die Lanthaniden und Actiniden in die organische Phase extrahieren können. Bevorzugt wird in Schritt a) eine organische Lösung bereitgestellt, wie sie im DIAMEX-Prozess anfällt.

In einer weiteren bevorzugten Ausführung des erfindungsgemäßen Verfahrens enthält die wässrige Phase Salpetersäure. Im technischen Wiederaufarbeitungsprozess werden Brennstäbe zur weiteren Verarbeitung in Salpetersäure aufgelöst. Da die erfindungsgemäßen $SO_3$-Ph-BTP, $SO_3$-Ph-BTBP oder $SO_3$-Ph-BTTP, welche beim Verfahren als hydrophile Komplexbildner eingesetzt werden, einerseits eine gute Säurestabilität aufweisen und andererseits in Salpetersäure wirksam sind, ist deren Einsatz unter den technischen Vorraussetzungen ohne Einsatz von Puffersubstanzen oder Neutralsalzen möglich.

Zur Extraktion in Schritt c) und zur Abtrennung im Schritt d) des erfindungsgemäßen Verfahrens können sämtliche dem Fachmann zur Verfügung stehenden Verfahren verwendet werden.

Die Erfindung wird im Folgenden anhand der Beispiele und der Figuren erläutert.

**Fig. 1** Schematischer Prozessverlauf im DIAMEX-SANEX-Prozess nach dem Stand der Technik.

**Fig. 2** Schematischer Prozessverlauf bei der erfindungsgemäßen Rückextraktion der Actiniden.

In Fig. 1 ist der in Europa vorgesehene Prozess der Wiederaufarbeitung von Kernbrennstäben nach dem DIAMEX-SANEX-Verfahren dargestellt. Es ist zu erkennen, dass nach der Uran-und Plutonium-Extraktion (PUREX) ein wässriges salpetersaures Raffinat erhalten wird (Durch Adaption des PUREX-Prozesses kann auch zusätzlich Neptunium extrahiert werden). Dieses Raffinat enthält neben Lanthaniden und leichteren Spaltprodukten die minoren Actiniden, hauptsächlich Americium und Curium. Lanthaniden und Actiniden werden im DIAMEX-Prozess zunächst gemeinsam mittels unspezifischen Komplexbildnern, wie TODGA oder D2EHPA, in eine organische Phase extrahiert. Die übrigen Spaltprodukte verbleiben in wässriger Lösung. Als zweiter Schritt im DIAMEX-Prozess werden die Lanthaniden und Actiniden in eine neue wässrige Phase zurückextrahiert, bevor sie der selektiven Actiniden Extraktion (SANEX) zugeführt werden.

In Fig. 2 ist nun dargestellt, in wiefern das aktuelle Verfahren mittels den erfindungsgemäßen Komplexbildnern

$SO_3$-Ph-BTP, $SO_3$-Ph-BTBP bzw. $SO_3$-Ph-BTTP vereinfacht werden kann. Da diese Komplexbildner wasserlöslich und säurestabil sind und darüber hinaus die Actiniden selektiv gegenüber den Lanthaniden komplexieren, kann der DIAMEX-Prozess abgekürzt werden. Anstatt der gemeinsamen Rückextraktion in wässriges Milieu, werden nur die Actiniden in Anwesenheit der erfindungsgemäßen Komplexbildner $SO_3$-Ph-BTP, $SO_3$-Ph-BTBP bzw. $SO_3$-Ph-BTTP selektiv rückextrahiert. Diese selektive Rückextraktion kann in saurer Lösung stattfinden, ohne dass Neutralsalze oder Puffersubstanzen hinzugefügt werden müssen.

Die Lanthaniden verbleiben in der organischen Phase und können abgetrennt werden. Ein aufwändiges SANEX-Verfahren wird somit überflüssig, was mit Kosteneinsparungen verbunden ist. Die $SO_3$-Ph-BTP, $SO_3$-Ph-BTBP und $SO_3$-Ph-BTTP können entweder zurückgewonnen oder thermisch entsorgt werden.

## Synthesen

Zunächst wurde nach literaturbekannten Vorschriften 2,6-Bis(5,6-diphenyl-1,2,4-triazin-3-yl)pyridin bzw. 6,6'-Bis(5,6-diphenyl-1,2,4-triazin-3-yl)-2,2'-bipyridin synthetisiert.

## Beispiel 1: Darstellung von $SO_3$-Ph-BTP

6 mL Oleum wurden auf 100 °C erhitzt. Dazu wurde langsam 1 g (1.85 mmol) 2,6-Bis(5,6-diphenyl-1,2,4-triazin-3-yl)pyridin zugegeben und 15 min. auf 170 °C erhitzt. Es wurde 3 Std. bei 120 °C gerührt, zwei Tage bei Raumtemperatur stehengelassen und anschließend einige Minuten auf 170 °C erhitzt. Es wurde auf 150 °C abgekühlt und so lange Argon eingeblasen bis keine weißen Dämpfe mehr entstanden. Die honigartige Masse wurde auf ca. 50 °C abgekühlt. Es wurde langsam gesättigte NaCl-Lösung zugegeben bis kein Schaum mehr gebildet wurde. Es wurde wenig Wasser zugegeben und die Reaktionsmischung mit konzentrierter NaOH-Lösung basisch gemacht. Anschließend wurde mit HCl-Lösung leicht angesäuert und die Flüssigkeit abgedampft.

Der Abdampfrückstand wurde 3-mal mit 25 mL Methanol ausgekocht. Der ausgekochte Rückstand (weißes Salz) wurde verworfen. Die Methanolphase wurde filtriert und das Filtrat eingedampft. Der Rückstand wurde noch 2-mal mit Methanol ausgekocht und eingedampft.

Graugrüner Feststoff, Ausbeute 1.25 g (78.5 %), Schmelzpunkt >350 °C. Löslichkeit in Wasser > 0.1 mol/L.

## Beispiel 2: Darstellung von $SO_3$-Ph-BTBP

$SO_3$-Ph-BTBP wurde aus 6,6'-Bis(5,6-diphenyl-1,2,4-triazin-3-yl)-2,2'-bipyridin analog zu $SO_3$-Ph-BTP hergestellt. Löslichkeit in Wasser > 0.1 mol/L.

## Extraktionsversuche

Es wurde der Einfluss von $SO_3$-Ph-BTP und $SO_3$-Ph-BTBP auf die Verteilungsverhältnisse von Am(III) und Eu (III) bei der Extraktion mit TODGA bzw. D2EHPA bestimmt. Aus allen Versuchen wurden die Verteilungsverhältnisse (Verhältnis der organischen zur wässrigen Konzentration) von Am(III) und Eu(III) mittels Gamma-Spektrometrie bestimmt. Die Berechnung der Verteilungsverhältnisse $D_M$ ergibt sich aus den Zählraten des Gammazählers, welcher die radioaktiven $^{241}$Am (III) - und $^{152}$Eu(III) - Isotope in volumengleichen Proben der wässrigen bzw. der organischen Phase erfasst gem. der Gleichung $D_M=[M]_{org}/[M]_{aq}$, wobei die Konzentration des Metalls M aus [M]=Zählrate/Volumen berechnet wird.

$SO_3$-Ph-BTP und $SO_3$-Ph-BTBP zeigten sich als besonders geeignet zur Komplexierung von Americium(III) (als Vertreter der Actiniden) in Salpetersäure, welche selektiv gegenüber Europium(III) (als Vertreter der Lanthaniden) erfolgt. Dabei steht die wässrige Komplexierung gemäß Gleichung 1 bzw. Gleichung 2 mit der Extraktion gemäß Gleichung 3 bzw. 4, welche mit einem gering selektiven Extraktionsmittel wie z.B. TODGA (*N,N,N',N'*-Tetra-n-octyl-diglycolamid; Gleichung 3) oder D2EHPA (Di-(2-ethylhexyl)phosphorsäure; Gleichung 4) erfolgt, in Konkurrenz.

## Gleichungen:

$$(1)\quad M^{3+}_{aq} + n\ SO_3\text{-Ph-BTP}_{aq} \quad = \quad M(SO_3\text{-Ph-BTP})_n{}^{3+}_{aq}$$

$$(2)\quad M^{3+}_{aq} + n\ SO_3\text{-}Ph\text{-}BTBP_{aq} = M(SO_3\text{-}Ph\text{-}BTBP)_n{}^{3+}_{aq}$$

$$(3)\quad M^{3+}_{aq} + 3\ NO_3{}^-_{aq} + 3\ TODGA_{org} = M(NO_3)_3TODGA_{3\ org}$$

$$(4)\quad M^{3+}_{aq} + 3\ H\text{-}D2EHPA_{org} = M(D2EHPA)_{3\ org} + 3\ H^+_{aq}$$

Da $SO_3$-Ph-BTP und $SO_3$-Ph-BTBP Americium(III) deutlich stärker komplexieren als Europium(III), wird letzteres nunmehr besser extrahiert als ersteres.

**Beispiel 3:**

Es wurden $^{291}$Am(III) und $^{152}$Eu(III) (je ca. 1 kBq/mL) aus 0.09 / 0.18 / 0.44 / 0.88 / 1.8 mol/L $HNO_3$ in eine organische Phase bestehend aus (0.2 mol/L TODGA + 5 % vol. 1-Octanol in Kerosin) extrahiert. Parallel zu diesem Versuch wurde eine Extraktion unter analogen Bedingungen durchgeführt mit dem Unterschied, dass wässrige Phase zusätzlich 10 mmol/L $SO_3$-Ph-BTP enthielt.

Tabelle 1 zeigt den Einfluss von $SO_3$-Ph-BTP auf die Extraktion von Am(III) und Eu(III) mit TODGA, einem solvatisierenden Extraktionsmittel (vgl. Gleichung 3). Ohne $SO_3$-Ph-BT werden sowohl Am(III) als auch Eu(III) extrahiert; die Verteilungsverhältnisse steigen gemäß Gleichung 3 mit der $HNO_3$-Konzentration an. Zugabe von $SO_3$-Ph-BTP unterdrückt die Extraktion von Am(III), während die Extraktion von Eu(III) wesentlich geringer beeinflusst wird. Dies ist auf die bevorzugte Komplexierung von Am(III) mit $SO_3$-Ph-BTP in der wässrigen Phase zurückzuführen. Unter den gegebenen experimentellen Bedingungen wird also aus ca. 0.1-0.5 mol/L $HNO_3$ Eu(III) extrahiert, während Am(III) hauptsächlich in der wässrigen Phase verbleibt. Der Trennfaktor $SF_{Eu(III)/Am(III)} = D_{Eu(III)} / D_{Am(III)}$ liegt im Bereich von 500-1000, während er ohne $SO_3$-Ph-BTP etwa bei 7 liegt.

**Tabelle 1.** Extraktion von Am(III) und Eu(III) mit TODGA, Einfluss der Zugabe von 10 mmol/L $SO_3$-Ph-BTP zur wässrigen Phase. Organische Phase: 0.2 mol/L TODGA + 5 % vol. 1-Octanol in Kerosin. Wässrige Phase: $^{241}$Am (III) + $^{152}$Eu(III) in $HNO_3$

| $[HNO_3]$ [mol/L] | $D_{Am(III)}$ $[SO_3$-Ph-TP] mmol/L | | $D_{Eu(III)}$ $[SO_3$-Ph-BTP] mmol/L | | $SF_{Eu(III)/Am(III)}$ $[SO_3$-Ph-TP] mmol/L | |
|---|---|---|---|---|---|---|
| | 0 | 10 | 0 | 10 | 0 | 10 |
| 0,09 | 3.3 | $1.1\cdot10^{-3}$ | 23.4 | 1.2 | 7.1 | 1100 |
| 0,18 | 12.4 | 0.014 | 89 | 11.9 | 7.2 | 850 |
| 0,44 | 83 | 0.35 | 390 | 180 | 4.7 | 510 |
| 0,88 | 530 | 4.5 | $> 10^3$ | $> 10^3$ | - | > 220 |
| 1,8 | 2100 | 90 | $> 10^3$ | $> 10^3$ | - | > 11 |

**Beispiel 4:**

Es wurden $^{241}$Am(III) und $^{152}$Eu(III) (je ca. 1 kBq/mL) aus 0.045 / 0.09 / 0.18 / 0.44 mol/L $HNO_3$ in eine organische Phase bestehend aus (0.1 mol/L D2EHPA in Kerosin) extrahiert. Die wässrige Phase enthielt entweder 0 oder 10 mmol/L $SO_3$-Ph-BTP

Tabelle 2 zeigt den Einfluss von $SO_3$-Ph-BTP auf die Extraktion von Am(III) und Eu(III) mit D2EHPA, einem flüssigen Kationenaustauscher (vgl. Gleichung 4). Während bei niedriger Säurestärke in Abwesenheit von $SO_3$-Ph-BTP sowohl Am(III) als auch Eu(III) extrahiert werden, unterdrückt $SO_3$-Ph-BTP die Extraktion von Am(III) deutlich stärker als die von Eu(III); der Trennfaktor betrug $SF_{Eu}$(III)/Am(III) > 3000.

**Tabelle 2.** Extraktion von Am(III) und Eu(III) mit D2EHPA, Einfluss der Zugabe von $SO_3$-Ph-BTP zur wässrigen Phase. Organische Phase: 0.1 mol/L D2EHPA in Kerosin. Wässrige Phase: $^{241}$Am(III) + $^{152}$Eu(III) in $HNO_3$.

| $[HNO_3]$ [mol/L] | $D_{Am(III)}$ $[SO_3$-Ph-BTP] mmol/L | | $D_{Eu(III)}$ $[SO_3$-Ph-BTP] mmol/L | | $SF_{Eu(III)/Am(III)}$ $[SO_3$-Ph-BTP] mmol/L | |
|---|---|---|---|---|---|---|
| | 0 | 10 | 0 | 10 | 0 | 10 |
| 0.045 | 14.7 | $1.10^{-3}$ | 380 | 5.3 | 26 | 5200 |
| 0.09 | 1.4 | $4.10^{-4}$ | 59 | 1.4 | 42 | 3400 |
| 0.18 | 0.14 | $5.10^{-5}$ | 6.0 | 0.37 | 44 | 7000 |
| 0.44 | $6.10^{-3}$ | < *d.l.* | 0.23 | 0.06 | 37 | - |

**Beispiel 5:**

Einem inaktiven Simulat eines PUREX-Raffinats wurden $^{241}$Am(III) und $^{l52}$Eu(III) (je ca. 1 kBq/mL) zugegeben. Es wurde in eine organische Phase bestehend aus (0.2 mol/L TODGA + 5 % vol. 1-Octanol in Kerosin) extrahiert. Ein Aliquot der beladenen organischen Phase wurde in Wasser rückextrahiert, ein anderes Aliquot in eine Lösung von 10 mmol/L $SO_3$-Ph-BTP in Wasser.

Durch den entsprechenden Versuch in Beispiel 5 wurde folgendes gezeigt: Es wurden zunächst Am(III) und Eu(III) mit Verteilungsverhältnissen > 1000 aus der Simulatlösung extrahiert. Nach Rückextraktion mit Wasser betrugen die Verteilungsverhältnisse $D_{AM(III)}$ = 23 und $D_{Eu}(_{III})$ = 180, der Trennfaktor $SF_{Eu(III)/Am(III)}$ = 7.5. Nach Rückextraktion in eine Lösung von 10 mmol/L $SO_3$-Ph-BTP in Wasser betrugen die Verteilungsverhältnisse jedoch $D_{Am(III)}$ = 0.059 und $D_{Eu(III)}$ = 49, der Trennfaktor $SF_{EU(III)/Am(III)}$ = 830.

Dies bedeutet aus prozesstechnischer Sicht, dass auch, wie in einem entsprechenden Trennprozess vorgesehen, zunächst Am(III) und Eu(III) gemeinsam aus $HNO_3$ extrahiert werden können und anschließend Am(III) selektiv in eine Lösung bestehend aus $HNO_3$ und $SO_3$-Ph-BT rückextrahiert werden kann.

**Beispiel 6:**

Es wurden $^{241}$Am(III) und $^{152}$Eu(III) (je ca. 1 kBq/mL) aus 0.5 mol/L $HNO_3$ in eine organische Phase bestehend aus (0.2 mol/L TODGA + 5 % vol. 1-Octanol in Kerosin) extrahiert. Die wässrige Phase enthielt entweder 0 mmol/L oder 10 mmol/L $SO_3$-Ph-BTBP.

Die Ergebnisse für den Einfluss von $SO_3$-Ph-BTBP auf die Extraktion von Am(III) und Eu(III) mit TODGA sind in Tabelle 3 zusammengefasst. Während sowohl Am(III) als auch Eu(III) aus 0.5 mol/L $HNO_3$ extrahiert werden, unterdrückt die Zugabe von 10 mmol/L $SO_3$-Ph-BTBP zur wässrigen Phase die Extraktion von Am(III). Die Extraktion von Eu (III) wird kaum beeinflusst. Diese Ergebnisse sind vergleichbar mit den mit $SO_3$-Ph-BTP erzielten.

**Tabelle 3.** Extraktion von Am(III) und Eu(III) mit TODGA, Einfluss der Zugabe von $SO_3$-Ph-BTBP zur wässrigen Phase. Organische Phase: 0.2 mol/L TODGA + 5 % vol. 1-Octanol in Kerosin. Wässrige Phase: $^{241}$Am(III) + $^{152}$Eu (III) in 0.5 mol/L $HNO_3$.

| $[SO_3$-Ph-BTBP] | $D_{Am(III)}$ | $D_{Eu(III)}$ | $SF_{Eu(III)/Am(III)}$ |
|---|---|---|---|
| 0 | 120 | 900 | 7.5 |
| 10 mmol/L | 0.33 | 150 | 450 |

Mit den erfindungsgemäßen Verbindungen $SO_3$-Ph-BTP, $SO_3$-Ph-BTBP und $SO_3$-Ph-BTTP stehen hydrophile Komplexbildner zur Verfügung, welche Actiniden bevorzugt gegenüber den Lanthaniden erstmalig auch in stark salpetersauren Lösungen komplexieren, ohne dass Neutralsalze oder Puffersubstanzen hinzugefügt werden müssen.

**Abkürzungen:**

| | |
|---|---|
| PUREX | Plutonium-Uranium Recovery by Extraction |
| DIAMEX | Diamide Extraction |

(fortgesetzt)

| | |
|---|---|
| SANEX | Selective Actinide Extraction |
| BTP | 2,6-Bis(5,6-diphenyl-1,2,4-triazin-3-yl)pyridin |
| BTBP | 6,6'-Bis(5,6-diphenyl-1,2,4-triazin-3-yl)-2,2'-bipyridin |
| BTTP | 6,6"-Bis(5,6-di(phenyl)-1,2,4-triazin-3-yl)-2, 2':6',2"-terpyridine |
| $SO_3$-Ph-BTP | 2,6-Bis(5,6-di(sulfophenyl)-1,2,4-triazin-3-yl)pyridine |
| $SO_3$-Ph-BTBP | 6,6'-Bis(5, 6-di(sulfophenyl)-1, 2, 4-triazin-3-yl)-2,2'-bipyridine |
| $SO_3$-Ph-BTTP | 6,6"-Bis(5,6-di(sulfophenyl)-1,2,4-triazin-3-yl)-2,2':6',2"-terpyridine |
| TODGA | N,N,N',N'-Tetra-n-octyl-diglycolamid |
| D2EHPA | Di-(2-ethylhexyl)phosphorsäure |
| Ln | Lanthaniden |
| An | Actiniden |
| FP | Spaltprodukte außer Lanthaniden (fission pro-ducts) |
| $D_M$ | Verteilungsverhältnis $D_M=[M]_{org}/[M]_{aq}$, wobei [M]~Zählrate/Volumen |
| $SF_{Eu(III)/Am(III)}$ | Trennfaktor ($D_{Eu(III)}$ / $D_{AM(III)}$) |

## Patentansprüche

1. Komplexbildner enthaltend 2,6-Bis(5,6-di(sulfophenyl)-1,2,4-triazin-3-yl)pyridine, 6,6'-Bis(5,6-di(sulfophenyl)-1,2,4-triazin-3-yl)-2,2'-bipyridine und /oder 6,6"-Bis(5,6-di(sulfophenyl)-1,2,4-triazin-3-yl)-2,2':6',2"-terpyridine nach den allgemeinen Formeln (III), (IV), (V), (VI), (VII),

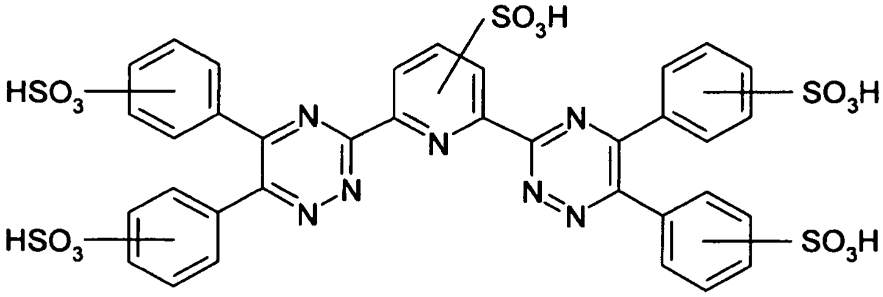

(III)

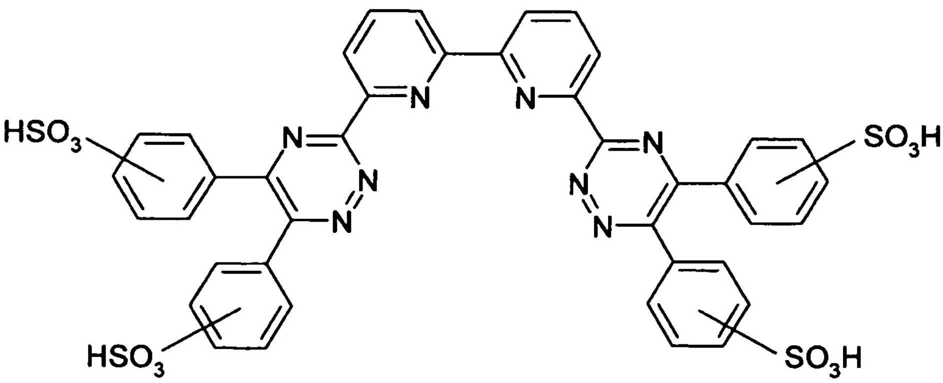

(IV)

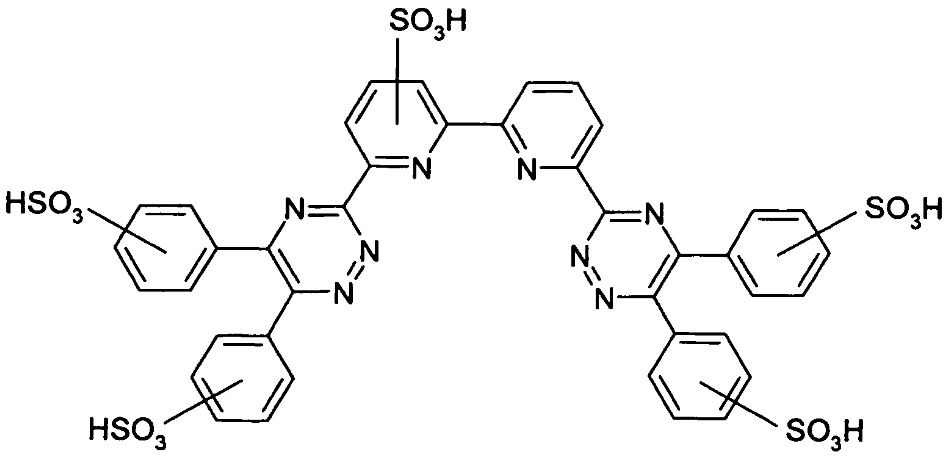

(V)

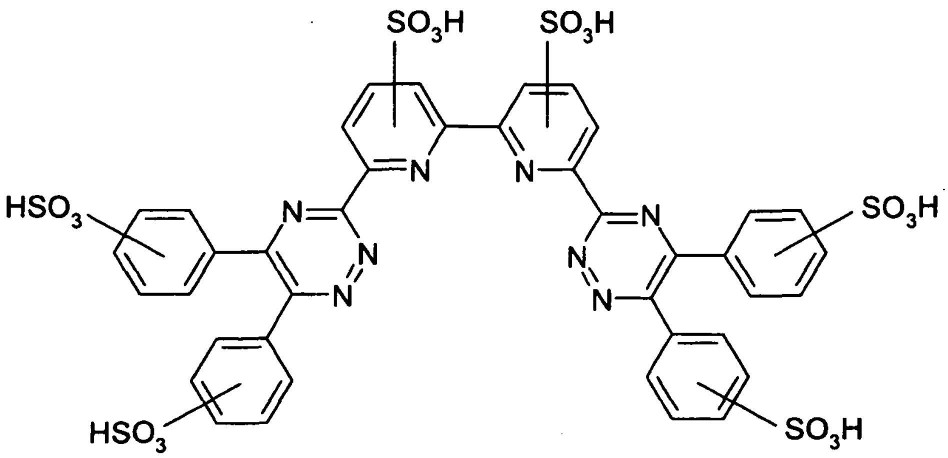

(VI)

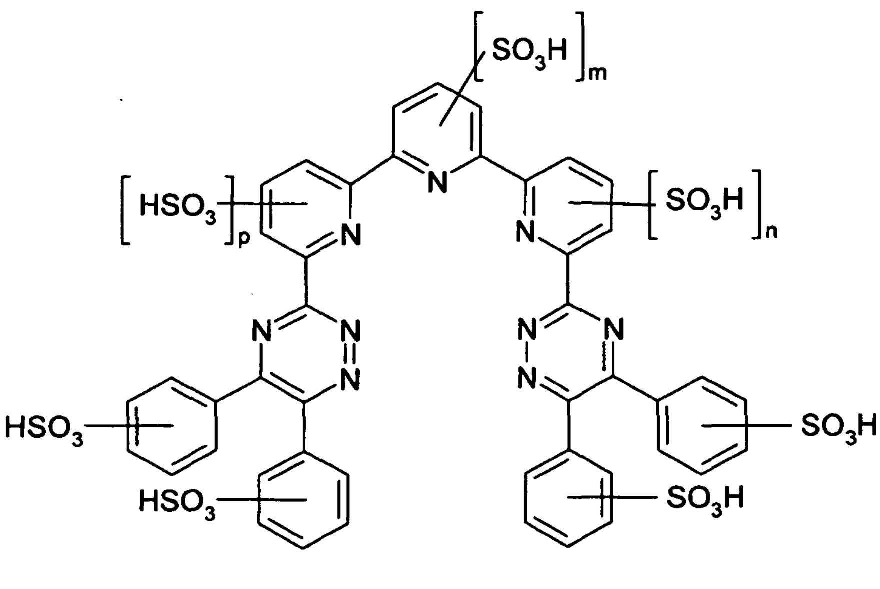

(VII)

wobei p,m,n = 0 oder 1 ist.

2. Moleküle nach Anspruch 1, wobei die $SO_3H$-Gruppen der jeweiligen Phenylgruppen in 4-Position (4-Sulfophenyl) sind.

3. Verfahren zur Herstellung von 2,6-Bis(5,6-di(sulfophenyl)-1,2,4-triazin-3-yl)pyridine, 6,6'-Bis(5,6-di(sulfophenyl)-1,2,4-triazin-3-yl)-2,2'-bipyridine und 6,6"-Bis(5,6-di(sulfophenyl)-1,2,4-triazin-3-yl)-2,2':6',2''-terpyridine nach Anspruch 1 oder 2 durch Sulfonierung von 2,6-Bis(5,6-diphenyl-1,2,4-triazin-3-yl)pyridin, 6,6'-Bis(5,6-diphenyl-1,2,4-triazin-3-yl)-2,2'-bipyridin oder 6,6''-Bis(5,6-diphenyl-1,2,4-triazin-3-yl)-2,2':6',2"-terpyridine mittels eines Sulfonierungsmittels aus der Gruppe Schwefelsäure, Oleum, Chlorsulfonsäure, Sulfamidsäure oder Schwefeltrioxid.

4. Verfahren nach Anspruch 3, wobei das Sulfonierungsmittel Oleum ist und die Umsetzung bei 100°C bis 170°C stattfindet.

5. Verfahren nach Anspruch 3 oder 4, wobei das Rohprodukt mit einem polaren Lösungsmittel, vorzugsweise Methanol, extrahiert wird.

6. Verwendung von Komplexbildnern enthaltend 2,6-Bis(5,6-di(sulfophenyl)-1,2,4-triazin-3-yl)pyridine, 6,6'-Bis(5,6-di (sulfophenyl)-1,2,4-triazin-3-yl)-2,2'-bipyridine und /oder 6,6"-Bis(5,6-di(sulfophenyl)-1,2,4-triazin-3-yl)-2,2':6',2"-terpyridine nach der allgemeinen Formel (I),

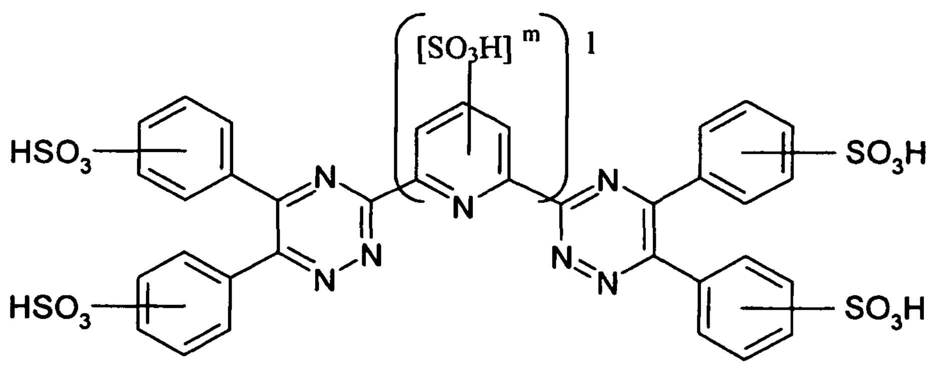

(I)

wobei l=1,2,3 und m=0,1 ist, zur selektiven Komplexierung von Actiniden in wässriger Lösung.

7. Verwendung nach Anspruch 6, wobei die Actiniden Plutonium, Americium, Curium und/oder Neptunium sind

8. Verfahren zur selektiven Extraktion von Actiniden umfassend folgende Verfahrensschritte:

a) Bereitstellen einer organischen Lösung enthaltend Lanthaniden und Actiniden, die mit Hilfe eines lipophilen Komplexierungsmittels gelöst sind,
b) Zugabe eines Komplexbildners enthaltend 2,6-Bis(5,6-di(sulfophenyl)-1,2,4-triazin-3-yl)pyridine, 6,6'-Bis(5,6-di(sulfophenyl)-1,2,4-triazin-3-yl)-2,2'-bipyridine und /oder 6,6"-Bis(5,6-di(sulfophenyl) - 1,2,4-triazin-3-yl)-2,2': 6',2"-terpyridine nach der allgemeinen Formel (I),

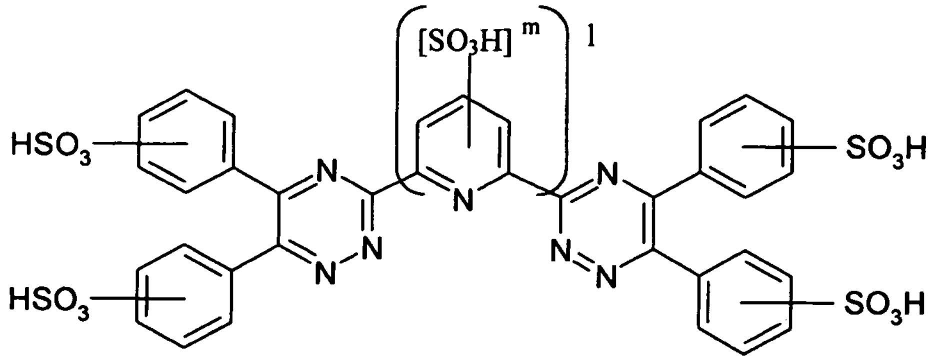

(I)

wobei l=1,2,3 und m=0,1 ist, in wässriger Lösung,
c) Rückextraktion der Actiniden in die wässrige Phase,
d) Abtrennen der wässrigen Phase.

9. Verfahren nach Anspruch 8, wobei die wässrige Lösung Salpetersäure enthält.

10. Verfahren nach Anspruch 8 oder 9, wobei die organische Lösung enthaltend Lanthaniden und Actiniden ein Extrakt der Wiederaufarbeitung von Kernbrennstoffen ist.

## Claims

1. A chelating agent containing 2,6-bis(5,6-di(sulfophenyl)-1,2,4-triazin-3-yl)pyridines, 6,6'-bis(5,6-di(sulfophenyl)-1,2,4-triazin-3-yl)-2,2'-bipyridines and/or 6,6"-bis(5,6-di(sulfophenyl)-1,2,4-triazin-3-yl)-2,2':6',2"-terpyridines in accordance with the general formulae (III), (IV), (V), (VI), (VII),

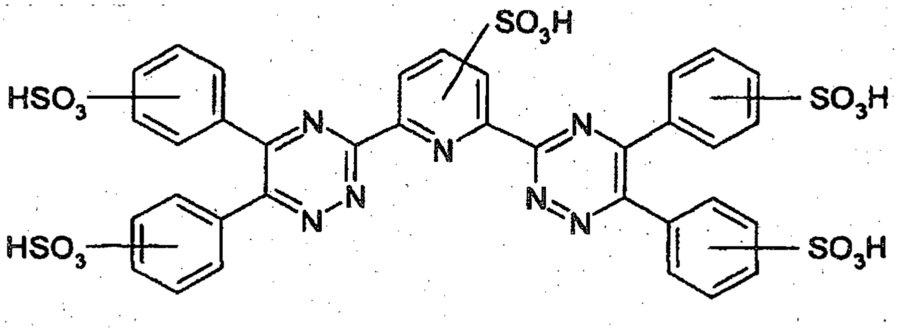

(III)

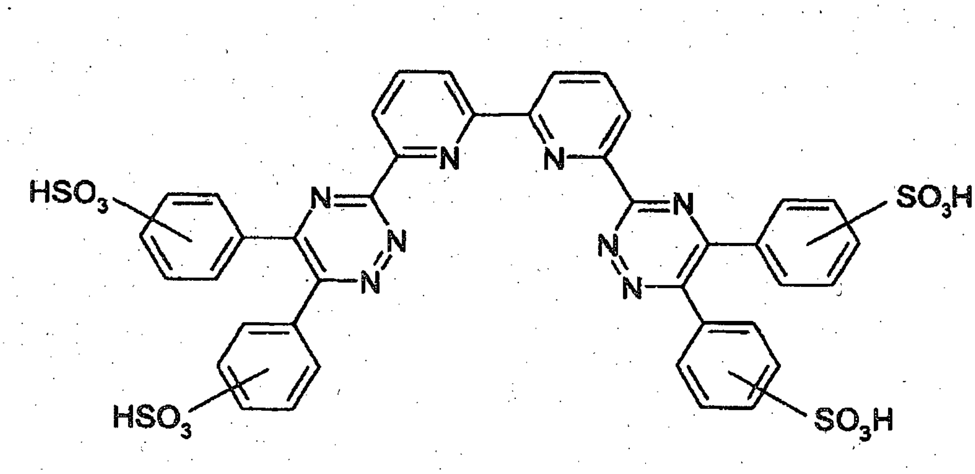

(IV)

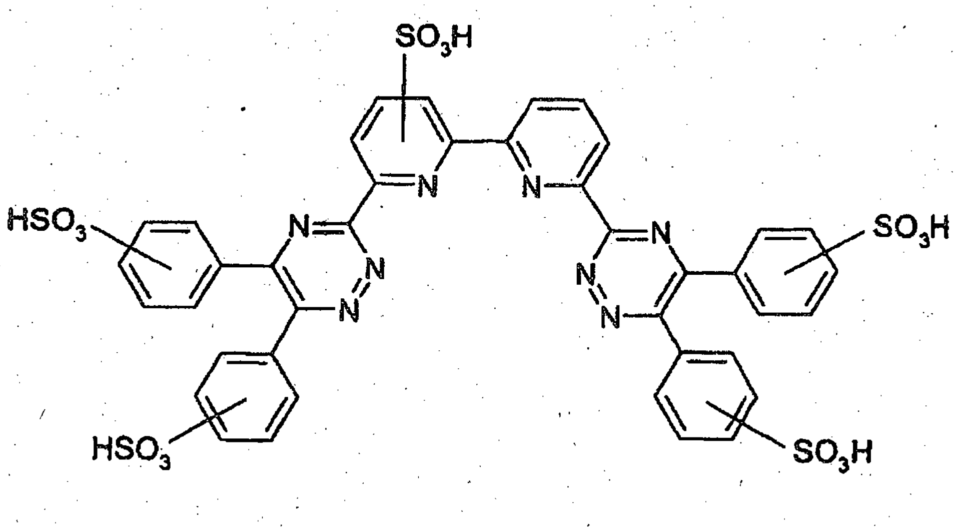

(V)

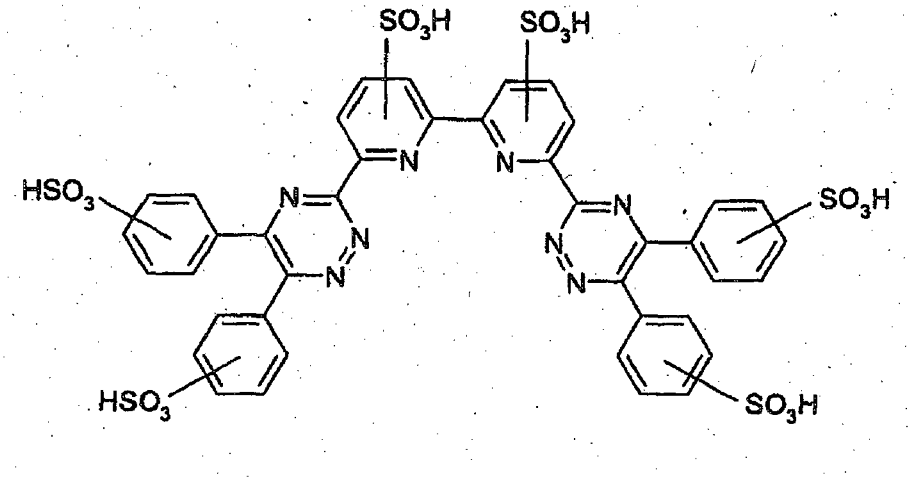

(VI)

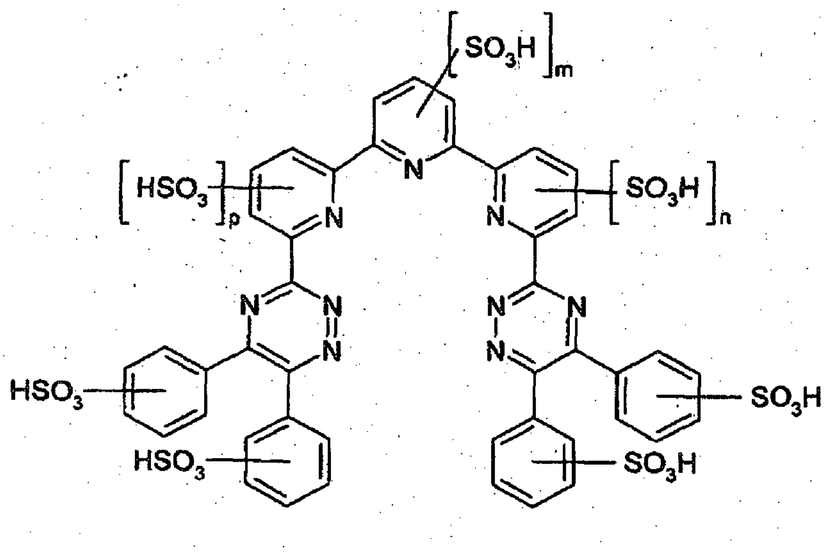

(VII)

wherein p,m,n=0 or 1.

**2.** The molecules according to claim 1, wherein the $SO_3H$ groups of the respective phenyl groups are in the 4-position (4-sulfophenyl).

3. A method for the preparation of 2,6-bis(5,6-di(sulfophenyl)-1,2,4-triazin-3-yl)pyridines, 6,6'-bis(5,6-di(sulfophenyl)-1,2,4-triazin-3-yl)-2,2'-bipyridines and 6,6"-bis(5,6-di(sulfophenyl)-1,2,4-triazin-3-yl)-2,2':6',2"-terpyridines according to claim 1 by sulfonation of 2,6-bis(5,6-diphenyl-1,2,4-triazin-3-yl)pyridine, 6,6'-bis(5,6-diphenyl-1,2,4-triazin-3-yl)-2,2'-bipyridine or 6,6"-bis(5,6-diphenyl-1,2,4-triazin-3-yl)-2,2':6',2"-terpyridines by means of a sulfonating agent from the group of sulfuric acid, oleum, chlorosulfuric acid, sulfamic acid or sulfur trioxide.

4. The method according to claim 3, wherein the sulfonating agent is oleum and the conversion takes place at 100°C to 170°C.

5. The method according to claim 3 or claim 4, wherein the raw product is extracted with a polar solvent, preferably methanol.

6. Use of chelating agents containing 2,6-bis(5,6-di(sulfophenyl)-1,2,4-triazin-3-yl)pyridines, 6,6'-bis(5,6-di(sulfophenyl)-1,2,4-triazin-3-yl)-2,2'-bipyridines and/or 6,6"-bis(5,6-di(sulfophenyl)-1,2,4-triazin-3-yl) -2,2':6',2"-terpyridines in accordance with the general formula (I),

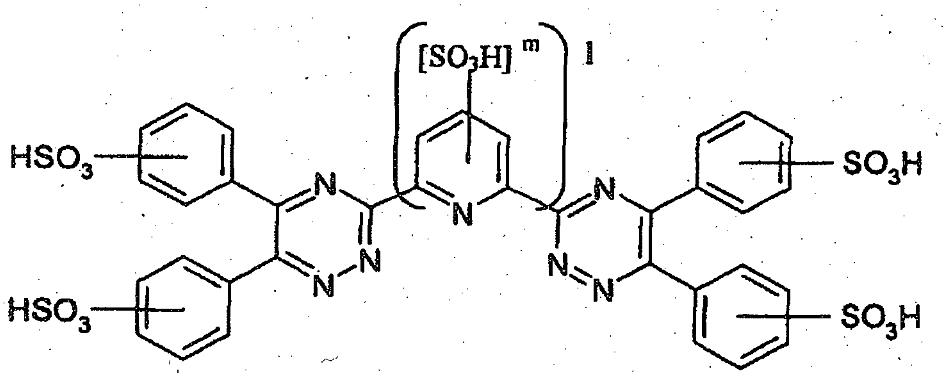

(I)

wherein l = 1, 2, 3 and m = 0, 1, for the selective chelation of actinides in aqueous solution

7. The use according to claim 6, wherein the actinides are plutonium, americium, curium and/or neptunium

8. A method for the selective extraction of actinides, comprising the following method steps:

(a) providing an organic solution containing lanthanides and actinides, which are dissolved with the aid of a lipophilic chelating agent,
(b) adding a chelating agent containing 2,6-bis(5,6-di(sulfophenyl)-1,2,4-triazin-3-yl)pyridines, 6,6'-bis(5,6-di (sulfophenyl)-1,2,4-triazin-3-yl)-2,2'-bipyridines and/or 6,6"-bis(5,6-di(sulfophenyl)-1,2,4-triazin-3-yl)-2,2':6',2"-terpyridines in accordance with the general formula (I),

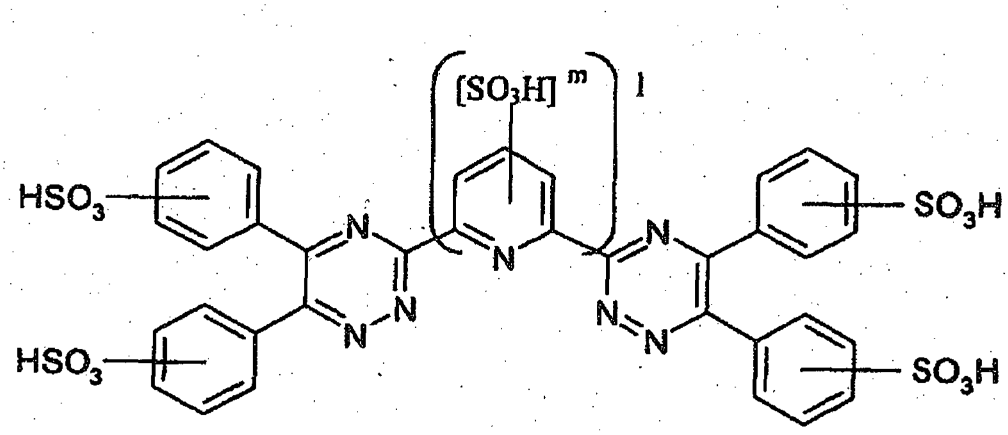

(I)

wherein l = 1, 2, 3 and m = 0, 1, in aqueous solution,
(c) back-extracting of the actinides into the aqueous phase,
(d) separating the aqueous phase.

**9.** The method according to claim 8, wherein the aqueous solution contains nitric acid.

**10.** The method according to claim 8 or claim 9, wherein the organic solution containing lanthanides and actinides is an extract from the reprocessing of nuclear fuels.

## Revendications

**1.** Agent complexant renfermant au moins l'un des composés suivants : 2,6-Bis(5,6-di(sulfophenyl)-1,2,4-triazin-3-yl) pyridine, 6,6'-Bis(5,6-di(sulfophenyl)-1,2,4- triazin-3-yl)-2,2'-bipyridine et/ou 6,6"-Bis(5,6-di(sulfophenyl)-1,2,4-triazin-3-yl)-2,2' ; 6',2"-terpyridine selon les formules (III), (IV), (V), (VI), (VII) ci-dessous :

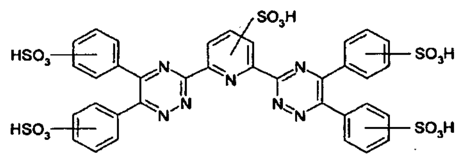

(III)

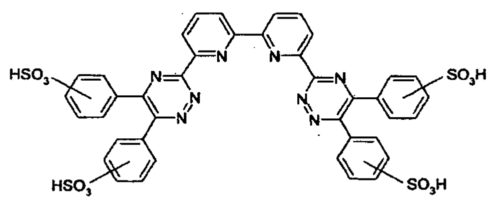

(IV)

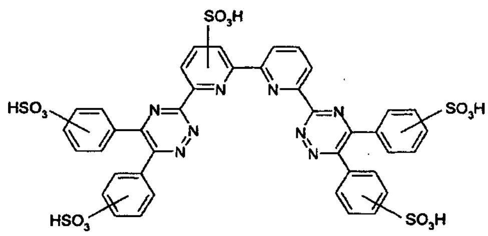

(V)

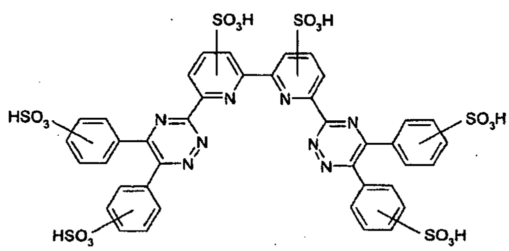

(VI)

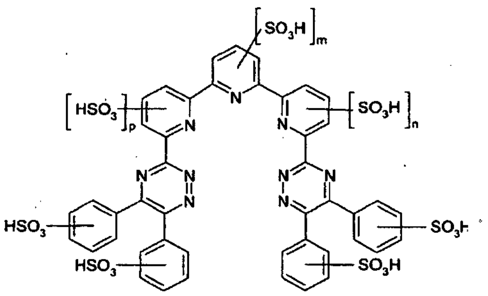

(VII)

dans lesquelles p,m,n = 0 ou 1.

2. Molécule conforme à la revendication 1, dans laquelle les groupes $SO_3H$ des groupes phényl respectifs sont des groupes (4-sulfophenyl) en position 4.

3. Procédé d'obtention de 2,6-Bis(5,6-di(sulfophenyl)-1,2,4-triazin-3-yl)pyridine, 6,6'-Bis(5,6-di(sulfophenyl)-1,2,4-triazin-3-yl)-2,2'-bipyri-dine et 6,6"-Bis(5,6-di(sulfophenyl)-1,2,4-triazin-3-yl)-2,2' : 6',2"-terpyridine conforme à la revendication 1 ou 2 par sulfonation de 2,6-Bis(5,6-diphenyl-1,2,4-triazin-3-yl)pyridin, 6,6'-Bis(5,6-diphenyl-1,2,4-triazin-3-yl)-2,2'-bipyridine et 6,6"-Bis(5,6-diphenyl-1,2,4-triazin-3-yl)-2,2' : 6',2"-terpyridine au moyen d'un agent de sulfonation choisi dans le groupe formé par l'acide sulfurique, l'acide sulfurique fumant, l'acide chlorosulfonique, l'acide sulfamique et le trioxyde de soude.

4. Procédé conforme à la revendication 3, selon lequel l'agent de sulfonation est de l'acide sulfurique fumant et la réaction a lieu entre 100°C et 170°C.

5. Procédé conforme à la revendication 3 ou 4, selon lequel le produit brut est extrait par un solvant polaire, de préférence le méthanol.

6. Utilisation d'agents complexants renfermant au moins l'un des composés suivants : 2,6-Bis(5,6-di(sulfophenyl)-1,2,4-triazin-3-yl)pyridine, 6,6'-Bis(5,6-di(sulfophenyl)-1,2,4-triazin-3-yl)-2,2'-bipyridine et/ou 6,6"-Bis(5,6-di(sulfophenyl)-1,2,4-triazin-3-yl)-2,2' : 6',2 "-terpyridine conformes à la formule générale (I) ci-dessous,

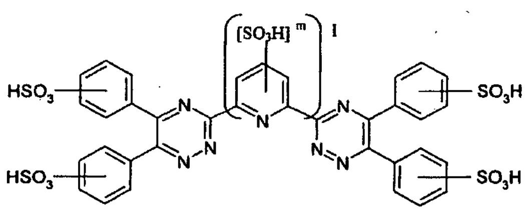

(I)

dans laquelle l = 1, 2, 3 et m = 0, 1, pour la complexation sélective d'actinides dans des solutions aqueuses.

7. Utilisation conforme à la revendication 6, selon laquelle les actinides sont le plutonium, l'americium, le curium et/ou le neptunium.

8. Procédé d'extraction sélection d'actinides comportant les étapes consistant à :

a) préparer une solution organique renfermant des lanthanides et des actinides qui sont dissouts à l'aide d'un agent de complexation lipophile,
b) addition d'un agent complexant renfermant au moins l'un des composés suivants : 2,6-Bis(5,6-di(sulfophenyl)-1,2,4-triazin-3-yl)pyridine, 6,6'-Bis(5,6-di(sulfophenyl)-1,2,4-triazin-3-yl)-2,2'-bipyridine et 6,6"-Bis(5,6-di(sulfophenyl)-1,2,4-triazin-3-yl)-2,2': 6',2"-terpyridine selon la formule générale (I),

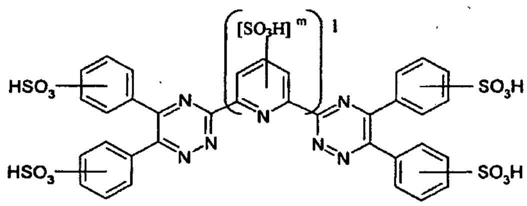

(I)

dans laquelle l = 1, 2, 3 et m = 0, 1, en solution aqueuse,
c) réextraction des actinides dans la phase aqueuse,
d) séparation de la phase aqueuse.

**9.** Procédé conforme à la revendication 8, selon lequel la solution aqueuse renferme de l'acide nitrique.

**10.** Procédé conforme à la revendication 8 ou 9, selon lequel la solution organique renfermant des lanthanides et des actinides est un extrait du retraitement de combustibles nucléaires.

Fig. 1

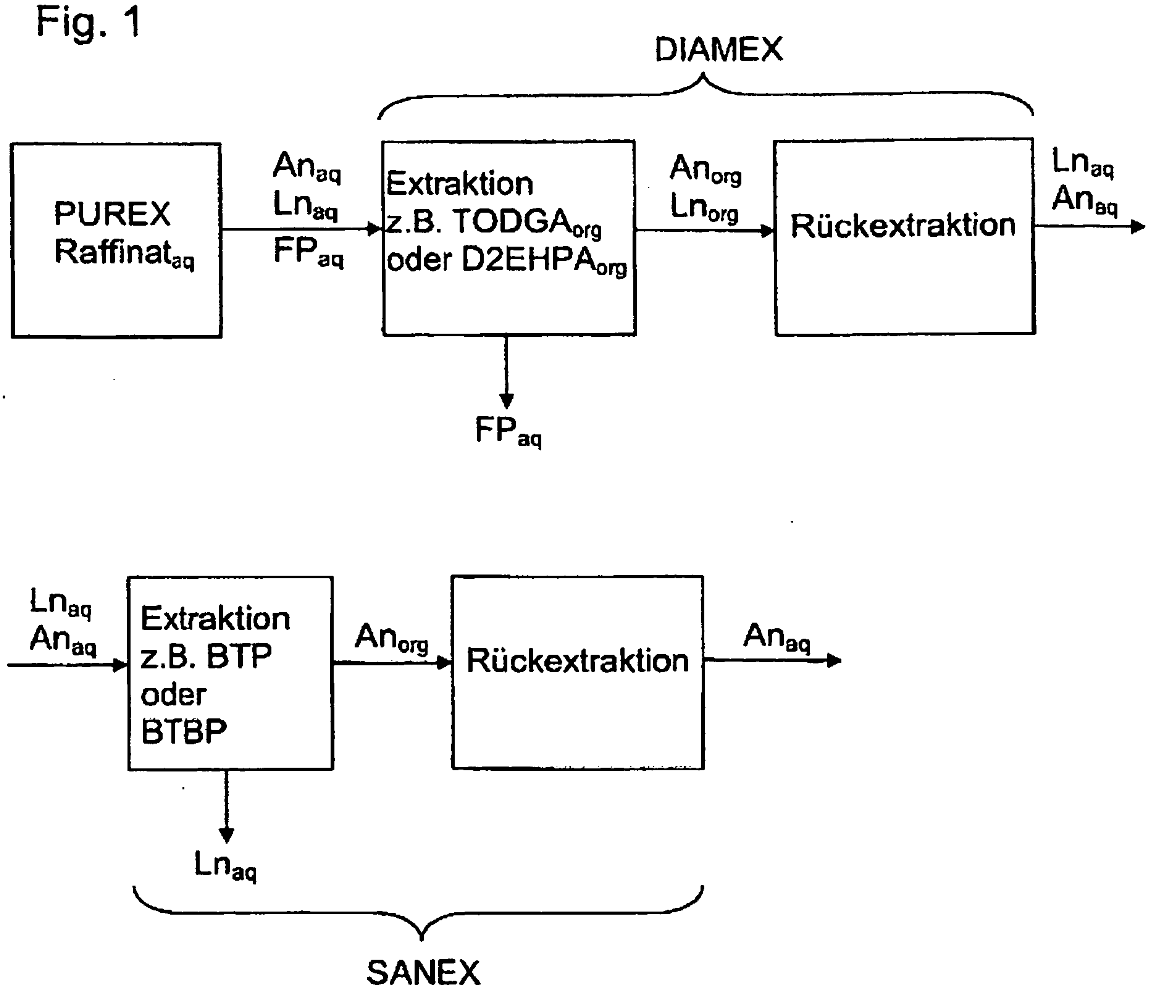
DIAMEX
PUREX $Raffinat_{aq}$
$An_{aq}$
$Ln_{aq}$
$FP_{aq}$
Extraktion z.B. $TODGA_{org}$ oder $D2EHPA_{org}$
$An_{org}$
$Ln_{org}$
Rückextraktion
$Ln_{aq}$
$An_{aq}$
$FP_{aq}$
$Ln_{aq}$
$An_{aq}$
Extraktion z.B. BTP oder BTBP
$An_{org}$
Rückextraktion
$An_{aq}$
$Ln_{aq}$
SANEX

Fig. 2

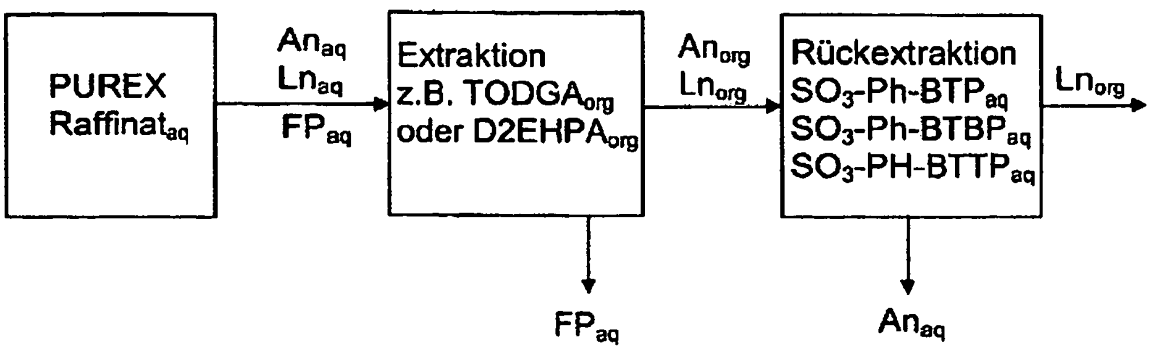
PUREX $Raffinat_{aq}$
$An_{aq}$
$Ln_{aq}$
$FP_{aq}$
Extraktion z.B. $TODGA_{org}$ oder $D2EHPA_{org}$
$An_{org}$
$Ln_{org}$
Rückextraktion
$SO_3$-Ph-$BTP_{aq}$
$SO_3$-Ph-$BTBP_{aq}$
$SO_3$-PH-$BTTP_{aq}$
$Ln_{org}$
$FP_{aq}$
$An_{aq}$

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 19810895 C1


### In der Beschreibung aufgeführte Nicht-Patentliteratur


- **A. GEIST ; C. HILL ; G. MODOLO ; M.R.ST.J. FOREMAN ; M. WEIGL ; K. GOMPPER ; M.J. HUDSON ; C. MADIC.** 6,6'-Bis(5,5,8,8-tetramethyl-5,6,7,8-tetrahydro-benzo-[1,2,4]triazin-3-yl)[2,2']bipyridine, an effective extracting agent for the separation of americium(III) and curium(III) from the lanthanides. *Solvent Extr. Ion Exch.,* 2006, vol. 24 (4), 463-483
- **G. MODOLO ; R. ODOJ.** Synergistic selective extraction of actinides(III) over lanthanides from nitric acid using new aromatic diorganyldithiophosphinic acids and neutral organophosphorus compounds. *Solvent Extr. Ion Exch.,* 1999, vol. 17 (1), 33-53
- TALSPEAK: a new method of separating americium and curium from the lanthanides by extraction from an aqueous solution of an aminopolyacetic acid complex with a monoacidic organophosphate or phosphonate. **B. WEAVER ; F.A. KAPPELMANN.** ORNL-3559. Oak Ridge National Laboratory, 1964
- **M. NILSSON ; K. NASH.** A Review of the development and operational characteristics of the TALSPEAK process. *Solvent Extr. Ion Exch.,* 2007, vol. 25 (6), 665-701
- **C. HERES et al.** *Proc. GLOBAL 2009,* September 2009
- **TRAISTER G L ; SCHILT A.** *Analytical Chemistry,* 1976, vol. 48 (8), 1216-1220